# EUROPEAN PATENT APPLICATION

(11) **EP 2 667 576 A1**
(43) Date of publication of application: **27.11.2013**
(21) Application number: 11856298.2
(22) Date of filing: 31.10.2011
(51) Int. Cl.: H04M 1/00, G06F 13/00, H04M 11/00

(54) **MOBILE COMMUNICATION DEVICE AND COMMUNICATION METHOD**

(30) Priority: 19.01.2011 JP 2011008559
(71) Applicant: NEC CASIO Mobile Communications, Ltd., Kawasaki-shi Kanagawa 211-8666 (JP)
(72) Inventor: UCHIDA, Kaoru, Kawasaki-shi Kanagawa 211-8666 (JP)
(74) Representative: Vossius & Partner
(86) International application number: PCT/JP2011/006101
(87) International publication number: WO 2012/098602

(57) **Abstract**

A mobile communication device and a communication method that enable remote monitoring of those who use electrical home appliances by collecting data from the electrical home appliances are provided without complicating the system configuration. The mobile communication device (10) includes a public network communication unit (20) that wirelessly communicates with a public network (60), a local area communication unit (30) that communicates with a sensor device (50) located in a neighbor area, and an access management unit (40) that accepts access from the sensor device (50) through the local area communication unit (30) and transmits sensor device usage information to an external device connected to the public network (60) based on information transmitted from the sensor device (50), or accepts access from the external device through the public network communication unit (20) and controls operation of the sensor device (50) in accordance with remote control information from the external device.

## Description

### Technical Field

The present invention relates to a mobile communication device and a communication method and, particularly, to a mobile communication device having a server function, and a communication method using the mobile communication device.

### Background Art

There are needs for distant family members to be able to keep an eye on the state of an elderly person living alone or check on the state of an elderly person living alone in the event of an emergency. For example, a service is offered that transmits the status of usage from a hot water dispenser used at the home of an elderly person to a system center and notifies distant family members about the status of usage of the hot water dispenser by email from the system center or allows distant family members to make inquiries to the system center about the status of usage of the hot water dispenser.

Further, in relation to remote control, a system capable of providing various services to an external device by installing a server program on a mobile phone terminal and running the server program in response to access from the external device is disclosed. Specifically, by running the server program on the mobile phone terminal according to an instruction from the external device, remote control of the mobile phone terminal is made by the external device.

In general, when providing the above-described service, electrical home appliances such as a hot water dispenser are connected to a system center of a service provider through a public network. Therefore, electrical home appliances need to have a communication function for a public network. The public network is an IP network or the like to implement internet connection, and it is a network provided by a telecommunications carrier, an internet service provider or the like, for example.

### Citation List

### Patent Literature

PTL1: Japanese Unexamined Patent Application Publication No. 2004-328578

### Summary of Invention

### Technical Problem

However, electrical home appliances used in the above-described service need to individually connect to a public network. Thus, it is necessary to make a communication contract for each of electrical home appliances used in the above-described service, and the base rate is charged for each communication contract. Thus, to avoid such charges, a system in which a concentrator that makes local connections with a plurality of electrical home appliances by near field communication or the like is installed at the home and connected to a public network is considered. However, in this case, the concentrator needs to have an interface compatible with the near field communication of electrical home appliances and make signal conversion by a modem or the like for a connection with a public network. There is thus a problem that the system configuration is complicated.

To solve the above problems, an exemplary object of the invention is to provide a mobile communication device and a communication method that enable remote monitoring of those who use electrical home appliances by collecting data from the electrical home appliances without complicating the system configuration.

### Solution to Problem

A mobile communication device according to a first exemplary aspect of the invention is a mobile communication device including a public network communication means for wirelessly communicating with a public network, a local area communication means for communicating with a sensor device located in a neighbor area, and an access management means for accepting access from the sensor device through the local area communication means and transmitting sensor device usage information to an external device connected to the public network based on information transmitted from the sensor device, or accepting access from the external device through the public network communication means and controlling operation of the sensor device in accordance with remote control information from the external device.

A communication method according to a second exemplary aspect of the invention is a communication method in a mobile communication device that communicates with an external device connected to a public network and a sensor device located in a neighbor area, the method including accepting access from the sensor device and transmitting sensor device usage information to the external device connected to the public network based on information transmitted from the sensor device, or accepting access from the external device and controlling operation of the sensor device in accordance with remote control information from the external device.

A sensor network system according to a third exemplary aspect of the invention is a sensor network system including an external device connected to a public network, a mobile communication device that communicates with the public network, and a sensor device located in a neighbor area of the mobile communication device, wherein the sensor device accesses the mobile communication device for transmitting detected sensor data, the external device accesses the mobile communication device for transmitting remote control information to remotely control the sensor device, and the mobile communication device accepts access from the sensor device and transmits sensor device usage information to the external device connected to the public network based on the sensor data transmitted from the sensor device, or accepts access from the external device through the public network communication means and controls operation of the sensor device in accordance with the remote control information from the external device.

### Advantageous Effects of Invention

According to the invention, it is possible to provide a mobile communication device and a communication method that enable remote monitoring of those who use electrical home appliances by collecting data from the electrical home appliances without complicating the system configuration.

### Brief Description of Drawings

Fig. 1 is a block diagram of a mobile communication device according to a first exemplary embodiment;
Fig. 2 is a block diagram of a mobile phone terminal according to the first exemplary embodiment;
Fig. 3 is a sequence of a remote control system according to the first exemplary embodiment; and
Fig. 4 is a sequence of a remote control system according to a second exemplary embodiment.

### Description of Embodiments

### (First exemplary embodiment)

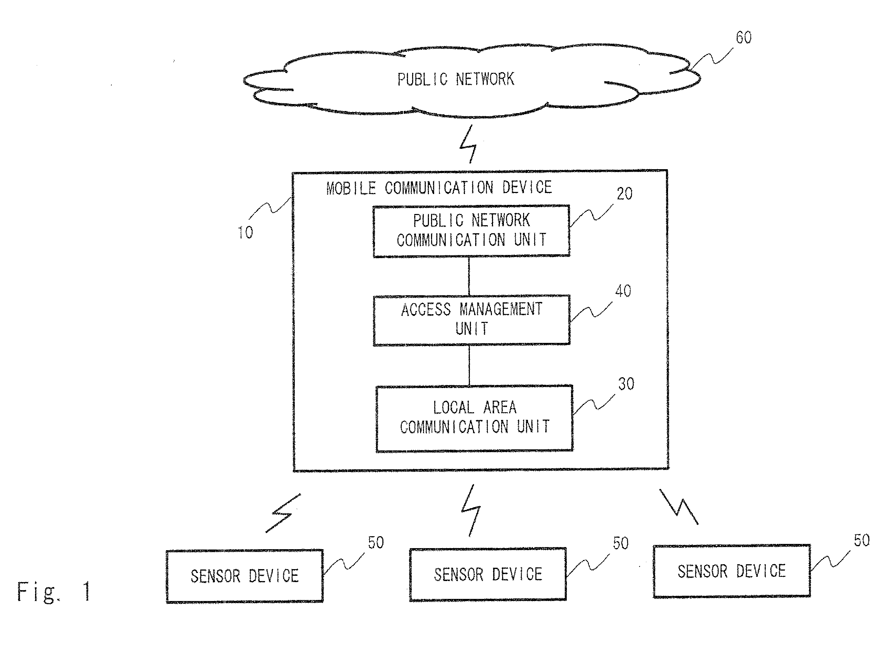

Exemplary embodiments of the present invention are described hereinafter with reference to the drawings. A configuration example of a mobile communication device according to a first exemplary embodiment of the invention is described with reference to Fig. 1. A mobile communication device 10 includes a public network communication unit 20, a local area communication unit 30, and an access management unit 40.

The mobile communication device 10 is a mobile phone terminal or a smartphone terminal, for example, which is a device with a communication function and easily portable.

The public network communication unit 20 connects to a radio network provided by a mobile telecommunications carrier, which is a so-called public network 60, and communicates with an external device or the like connected to the public network 60. Specifically, the public network communication unit 20 performs communication using predetermined wireless standards with a base station device installed by a mobile telecommunications carrier. Further, the public network communication unit 20 may perform communication with an external device or the like connected to the public network 60 using WiFi (Wireless Fidelity) for wireless LAN communications. In this case, the public network 60 performs communication using predetermined wireless standards with an AP (Access Point) installed by a wireless LAN service provider.

The local area communication unit 30 performs communication with a sensor device 50 located in a neighbor area. The local area communication unit 30 performs near field radio communication for connection with the sensor device 50. The near field radio communication is communication using infrared ray or communication using Bluetooth (registered trademark), Bluetooth Low Energy or the like, for example. In the case of using infrared ray, communication can be made with communication equipment at a distance of several tens of centimeters. In the case of using Bluetooth, communication can be made with communication equipment at a distance of several tens of meters. The neighbor area is a range where near field radio communication is available between the mobile communication device 10 and the sensor device 50.

The sensor device 50 detects a change of state and transmits a detection result to the mobile communication device 10. The sensor device 50 performs near field radio communication with the mobile communication device 10. The sensor device 50 is a hot water dispenser that includes a sensor for detecting the pressing of a button, for example. The hot water dispenser that serves as the sensor device 50 detects that a button such as a hot water supply button of the hot water dispenser is pressed and notifies that to the mobile communication device 10. Further, the sensor device 50 may be a refrigerator that includes a sensor for detecting the opening and closing of a door. The refrigerator that serves as the sensor device 50 may detect that the refrigerator door is opened or closed and notify that to the mobile communication device 10. Alternatively, the sensor device 50 may be a toilet that includes a sensor for detecting usage based on flushing, door opening and closing and the like. The toilet that serves as the sensor device 50 may detect the state of usage of the toilet and notify that to the mobile communication device 10. The sensor device 50 may detect various changes of state by a sensor built in familiar equipment or the like and notify those to the mobile communication device 10.

The access management unit 40 receives access from the sensor device 50 through the local area communication unit 30. Further, the access management unit 40 transmits sensor device usage information to an external device connected to the public network 50 based on information transmitted from the sensor device 50. The access management unit 40 may register information of the sensor devices 50 that can be connected to the mobile communication device 10 and permit access from the registered sensor devices 50 only. In this case, the access management unit 40 may register identifiers that uniquely identify the sensor devices 50. For example, the identifier may be a MAC address of the sensor device 50 or ID set by a user.

When the access management unit 40 receives a detection result about a change of state from the sensor device 50, it determines that the sensor device 50 is used and transmits sensor device usage information to an external device through the public network communication unit 20.

Further, the access management unit 40 receives access from an external device through the public network communication unit 20. The access management unit 40 controls the operation of the sensor device 50 according to remote control information from the external device. The access management unit 40 may register information of external devices that can be connected to the mobile communication device 10 and permit access from the registered external devices only. In this case, the access management unit 40 may register identifiers that uniquely identify external devices. For example, the identifier may be a MAC address of the external device, IP address, or ID set by a user.

Furthermore, the access management unit 40 may receive remote control information from an external device and control the timing to receive communication from the sensor device 50. For example, when the access management unit 40 receives remote control information indicating that the state of the sensor device 50 should be notified at regular intervals, the access management unit 40 may control the operation of the sensor device 50 so as to notify the state of the sensor device 50 on a regular basis, not only when the sensor device 50 has detected a change of state.

As described above, with use of the mobile communication device 10 shown in Fig. 1, it is possible to relay communication between the sensor devices 50 located in a local area and external devices connected to the public network 60 through the mobile communication device 10. Further, because communication between the mobile communication device 10 and the public network 60 is performed using the radio communication function originally incorporated in the mobile communication device 10, there is no need to install new equipment or the like and it is thus possible to relay communication between the external devices and the sensor devices 50 without complicating the system.

Further, an electrical home appliance that includes the sensor device 50 does not need to have a communication function to communicate with a public network. It is thus not necessary to make a communication contract to perform communication with a public network for each of electrical home appliances, which eliminates the need to pay the base rate or the like for the communication contract. Further, an electrical home appliance only needs to include the sensor device 50 and perform near field communication, downsizing and lower power consumption are possible compared with the case of incorporating a communication function for performing communication with the public network 60.

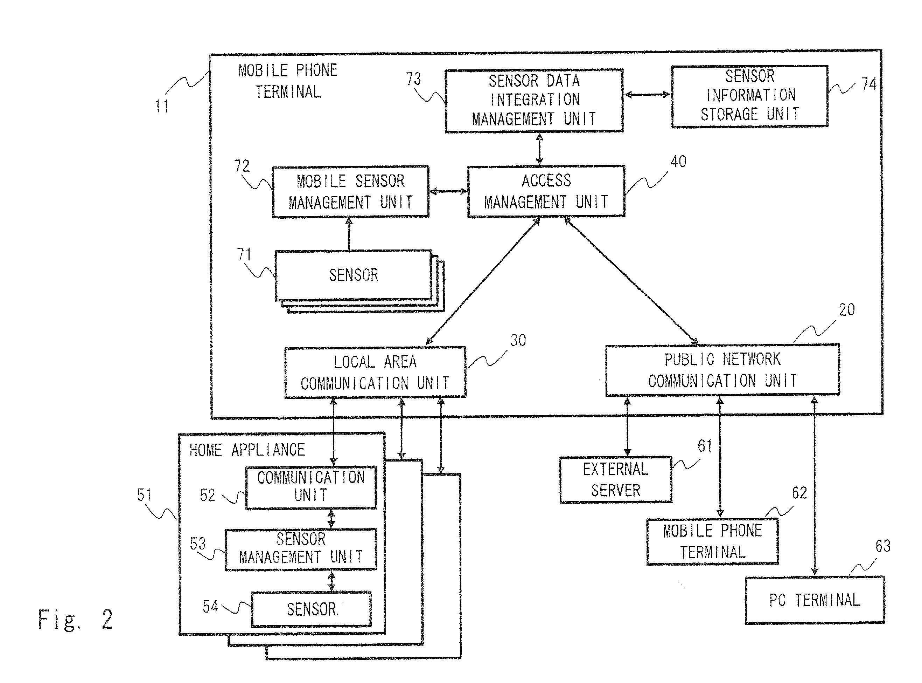

A configuration example of a mobile phone terminal 11 according to the first exemplary embodiment of the invention is described hereinafter with reference to Fig. 2. The mobile phone terminal 11 includes a public network communication unit 20, a local area communication unit 30, an access management unit 40, a sensor 71, a mobile sensor management unit 72, a sensor data integration management unit 73, and a sensor information storage unit 74. The public network communication unit 20, the local area communication unit 30 and the access management unit 40 are the same as those of Fig. 1 and not described in detail.

The sensor 71 is built in the mobile phone terminal 11 and detects a change of state of the mobile phone terminal 11. For example, the sensor 71 is a GPS device and can receive latitude and longitude information where the mobile phone terminal 11 is located through a GPS antenna (not shown). Further, the sensor 71 may be an acceleration sensor, a temperature sensor or the like, and the mobile phone terminal 11 may include a plurality of sensors.

The mobile sensor management unit 72 receives sensor data output from the sensor 71 and outputs the sensor data to the access management unit 40. Further, the mobile sensor management unit 72 controls the operation of the sensor 71 such as the timing to generate sensor data in the sensor 71. For example, when movement of a certain distance is detected by the sensor 71, the mobile sensor management unit 72 may cause the sensor 71 to generate sensor data and receive the generated sensor data. Further, when a certain change of temperature is detected by the sensor 71, the mobile sensor management unit 72 may cause the sensor 71 to generate sensor data and receive the generated sensor data.

The access management unit 40 has a Web server function (which is referred to hereinafter as a terminal server) and determines whether to allow connection to external devices such as an external server 61, a mobile phone terminal 62 and a PC terminal 63 that make access through the public network communication unit 20, an electrical home appliance 51 that makes access through the local area communication unit 30 and the like. The external devices, the electrical home appliance 51 and the like indicate URL set to the terminal server and make access to the mobile phone terminal 11. Further, the access management unit 40 outputs sensor data transmitted from the electrical home appliance 51 and sensor data detected by the sensor 71 built in the mobile phone terminal 11 to the sensor data integration management unit 73.

The sensor data integration management unit 73 integrates the received sensor data and generates information about the status of a person who has used the electrical home appliance 51. For example, the sensor data integration management unit 73 generates a situation judging result related to the state of a person who has used the electrical home appliance 51 using the sensor data about the status of usage of the electrical home appliance 51 detected by the electrical home appliance 51 operating as the sensor device 50 and geodetic history data with time stamps obtained by the sensor 71 having the GPS function.

A specific example of the situation judging result is described hereinafter. The sensor data integration management unit 73 determines whether the mobile phone terminal 11 is located at the home or not from the geodetic history information. When the sensor data integration management unit 73 detects that the electrical home appliance 51 is used in the state where the mobile phone terminal 11 is located within the home, it estimates that a person who holds the mobile phone terminal 11 and a person who has used the electrical home appliance 51 are the same and can thereby determine that the person is living a normal life. On the other hand, when the sensor data integration management unit 73 detects that the electrical home appliance 51 is used in the state where the mobile phone terminal 11 is located outside the home, it estimates that a person who holds the mobile phone terminal 11 and a person who has used the electrical home appliance 51 are different. It can be thus determined that there is a possibility that a person considered to be a suspicious individual has broken into the home while a person to be monitored is going out nearby.

In this manner, by combining the sensor data from the sensor 71 built in the mobile phone terminal 11 and the sensor data received from a sensor such as the electrical home appliance 51 outside the mobile phone terminal 11, it is possible to generate effective information that is unknowable from one sensor data only and present it to a person remotely monitoring a target. Further, the sensor data acquired from the sensor 71 and the electrical home appliance 51 or the data integrated by the sensor data integration management unit 73 (which is referred to hereinafter as integrated data) is stored into the sensor information storage unit 74.

A different example of the information generated by the sensor data integration management unit 73 is described hereinbelow. The sensor data integration management unit 73 receives information about the alarm time set to the mobile phone terminal 11 from the mobile phone terminal 11 and operation information of the electrical home appliance 51 from the electrical home appliance 51, for example. In this case, when it is not detected that the electrical home appliance 51 is used within a specified time period from the alarm time, the sensor data integration management unit 73 can estimate that a person to be monitored is in the situation of being unable to move due to illness, accident or the like. The sensor data integration management unit 73 notifies this estimated situation to the external server 61 or the like through the public network communication unit 20. It is thereby possible to manage the safety of a person living alone, for example.

Further, the sensor data integration management unit 73 receives information about the remaining amount of battery from the mobile phone terminal 11 and further receives operation information of the electrical home appliance 51 from the electrical home appliance 51. When the sensor data integration management unit 73 detects that the remaining battery is low, it may send a message suggesting to charge the mobile phone terminal 11 to the electrical home appliance 51 where the operation is detected. By sending a message to the electrical home appliance 51 being used, it is possible to reliably notify a user of the mobile phone terminal 11 of the message suggesting charging.

When a condition set to transmit sensor device usage information (including integrated data) to the external server 61, the mobile phone terminal 62, the PC terminal 63 or the like is met, the access management unit 40 transmits the sensor device usage information to the external device such as the external server 61, the mobile phone terminal 62 or the PC terminal 63 through the public network communication unit 20. The preset condition may be that the mobile phone terminal 11 is located near the home (i.e. a person to be monitored is not out for travel or the like), any of the hot water dispenser and the refrigerator is not used for one whole day or more, or the toilet is not used for a period of four hours or more (hereinafter as a condition 1), for example. Alternatively, the preset condition may be that the sensor device 50 has detected a change of state (hereinafter as a condition 2).

A configuration example of the electrical home appliance 51 is described hereinbelow. The electrical home appliance 51 includes a communication unit 52, a sensor management unit 53 and a sensor 54. The communication unit 52 performs near field communication with the mobile phone terminal 11. The sensor 54 is a sensor that detects a change of state. Specifically, the sensor 54 may be a GPS device, an acceleration sensor, a temperature sensor or the like as described above. The sensor management unit 53 receives a detection result of a change of state in the sensor 54 and outputs it to the communication unit 52. Further, the sensor management unit 53 receives description of control of the electrical home appliance 51 notified from the mobile phone terminal 11 and executes control on the sensor 54. As described above, the electrical home appliance 51 only includes the communication unit 52 that performs near field communication with the mobile phone terminal 11 and there is no need to have a function to perform communication with the public network 60. It is thus possible to reduce the cost for the sensor function part of the electrical home appliance 51 and achieve downsizing of the sensor function part.

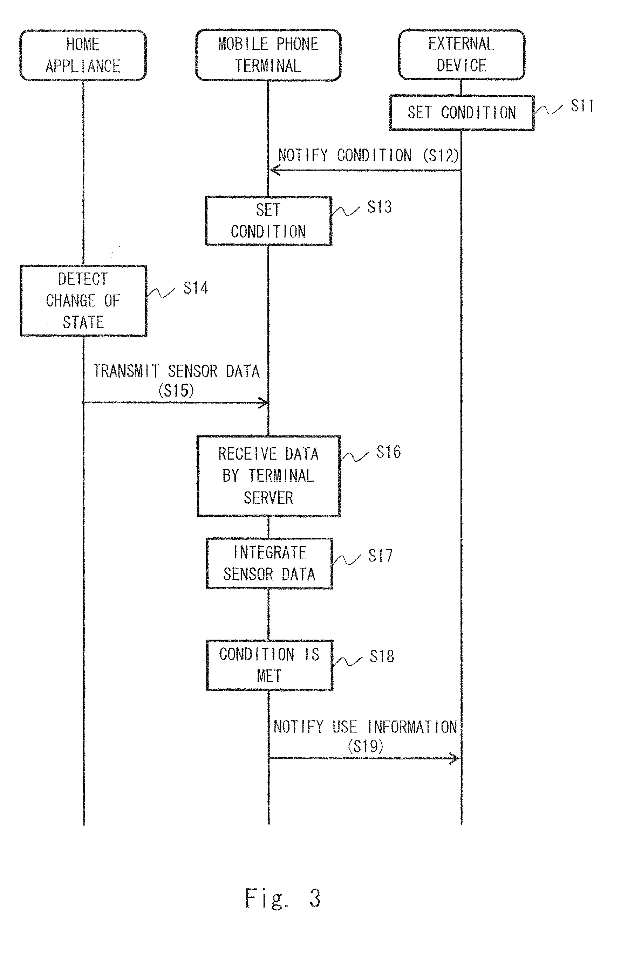

A flow of a process in a remote control system according to the first exemplary embodiment of the invention is described hereinafter with reference to Fig. 3. The external device such as the PC terminal 63 that is connected to the public network 60 sets a condition for receiving notification of the sensor device usage information (S11). The condition set in this step is the condition 1, the condition 2 or the like described above, or it may be a condition different from the conditions 1 and 2. Next, the external device notifies the set condition to the mobile phone terminal 11 (S12). The mobile phone terminal 11 reeeives the condition set by the external device through the public network communication unit 20.

Then, the access management unit 40 in the mobile phone terminal 11 sets the condition received from the external device (S13). After that, the electrical home appliance 51 that serves as the sensor device 50 detects a change of state (S14). The electrical home appliance 51 then transmits sensor data indicating the detection of a change of state to the mobile phone terminal 11 (S15). The electrical home appliance 51 transmits an API execution instruction indicating that the detection result of a change of state is transmitted to the external device through the public network communication unit 20 to the mobile phone terminal 11. The access management unit 40, or the terminal server, receives the sensor data through the local area communication unit 30 (S16). The access management unit 40 outputs the received sensor data to the sensor data integration management unit 73 and further outputs the sensor data detected in the sensor 71 built in the mobile phone terminal 11 to the sensor data integration management unit 73.

Then, the sensor data integration management unit 73 integrates the sensor data using the received sensor data (S17). Specifically, the sensor data integration management unit 73 generates a situation judging result related to the state of a person who has used the electrical home appliance 51. Next, the access management unit 40 determines whether the condition that is set in Step S13 is met or not (S18). When the preset condition is met, the access management unit 40 executes the API and notifies the sensor device usage information to the external device (S19). When, on the other hand, the preset condition is not met, the access management unit 40 stores the sensor device usage information into the sensor information storage unit 74 and notifies the sensor device usage information to the external device when the condition is met.

As described above, with use of the mobile phone terminal 11 according to the first exemplary embodiment of the invention, integrated data can be generated by combining sensor data received from the external device located in the neighbor area and sensor data detected by the sensor built in the mobile phone terminal 11. The mobile phone terminal 11 can thereby generate effective information that is unknowable from the sensor data received from the external device only and present it to a person who is remotely monitoring a person at the home.

Further, the mobile phone terminal 11 includes the terminal server. Therefore, an external device or an electrical home appliance serving as the sensor device can access the terminal server using URL set to the terminal server in the mobile phone terminal 11. Thus, the sensor device 50 can actively access the mobile phone terminal 11 and let it notify the sensor device usage information to the external device before receiving an instruction about a request for transmission of sensor data from the mobile phone terminal 11. Further, the external device can also actively access the mobile phone terminal 11 and control the sensor device 50.

As one aspect of the service using the mobile phone terminal 11 according to the first exemplary embodiment of the invention, family members living in a distant area can receive usage information of the sensor device 50 or control the operation of the sensor device 50 through the mobile phone terminal 11 of an elderly person living alone or the like. As another aspect of the service, when an administrative agency, a medical center, an elderly care service provider or the like provides a service to keep an eye on an elderly person living alone, for example, they can access the mobile phone terminal 11 of the elderly person living alone and thereby implement the service.

Further, an external service company such as a company that provides safety and security monitoring services connects a server in its company to a mobile terminal of each user and can thereby provide the safety and security monitoring services.

### (Second exemplary embodiment)

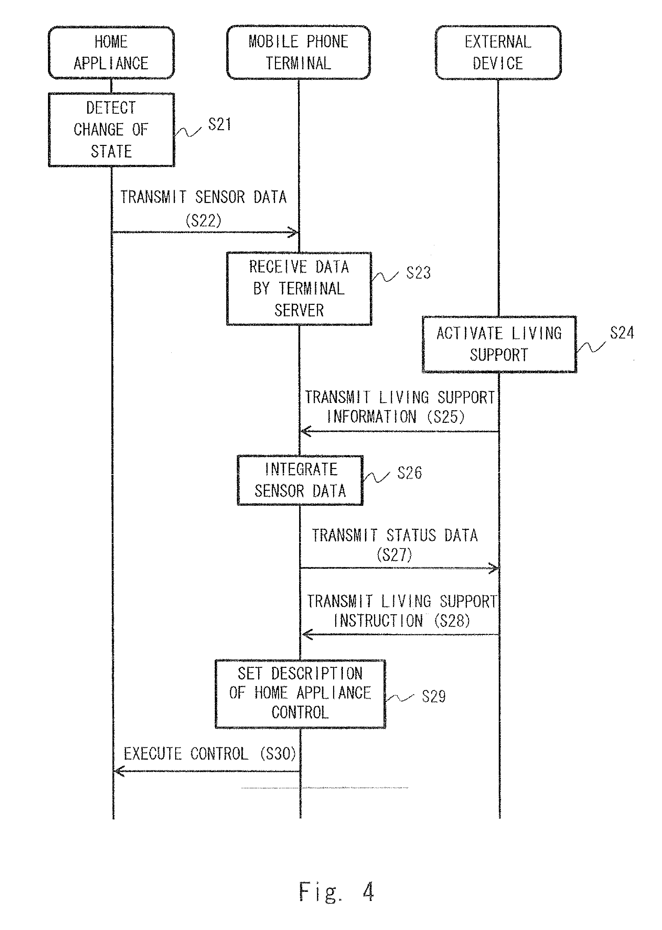

A flow of a process in a remote control system according to the second exemplary embodiment of the invention is described hereinafter with reference to Fig. 4. The configuration of the mobile phone terminal 11 is the same as that of Fig. 2. In the remote control system in Fig. 4, an external device such as a server or a PC terminal acts as the starting point and reads sensor data of a sensor in the mobile phone terminal 11 or an external device connected with the mobile phone terminal 11 using near field communication. For example, a flow of a process in the remote control system in the case where "there is a concern about the body temperature and water intake of a family member living in a distant area due to the hot weather" is described below.

First, the electrical home appliance 51 at the home detects a change of state or the current state by a sensor included therein (S21) and transmits sensor data to the mobile phone terminal 11 (S22). The sensor data transmitted to the mobile phone terminal 11 may be the room temperature, humidity, water server usage data, the number of times of using a refrigerator, the on/off status of lighting in the home and the like, for example. Further, the mobile phone terminal 11 can receive various types of sensor data by incorporating a sensor into familiar electrical home appliances.

Next, the mobile phone terminal 11 receives sensor data by the terminal server of the access management unit 40 (S23). The access management unit 40 may store the received sensor data into an arbitrary memory device (RAM, ROM etc.) in the mobile phone terminal 11 or the sensor information storage unit 74.

Then, the external device activates an application for living support (S24). The application for living support is an application used for giving advice to a family member living in a distant place or controlling the electrical home appliance 51 or the like through the mobile phone terminal 11 using sensor information about the distant family member or the like.

After that, the external device transmits living support information to the mobile phone terminal 11 (S25). The external device transmits an API execution instruction indicating collection of sensor data related to the transmitted living support information, for example. The living support information is information such as "there is a concern about the body temperature and water intake of a family member living in a distant area due to the hot weather" as described above, for example. Several items of such living support information may be set in advance by the application, and a user of the external device may set desired living support information from those several items.

Then, the sensor data integration management unit 73 in the mobile phone terminal 11 integrates sensor data (S26). For example, when the sensor data integration management unit 73 receives the living support information indicating that "there is a concern about the body temperature and water intake of a family member living in a distant area due to the hot weather" from the external device, it collects the room temperature, humidity, water server usage data, health care equipment data or the like from the memory device, the sensor information storage unit 74 or the like. In other words, the sensor data integration management unit 73 collects the sensor data related to the living support information. The living support information and the sensor data required for the living support information may be associated with each other in advance. The health care equipment data is data of the body temperature measured by a user using a thermometer, the blood pressure measured using a blood pressure meter or the like, for example. By incorporating a sensor into a thermometer, a blood pressure meter or the like, the mobile phone terminal 11 can receive the health care equipment data from those equipment. The access management unit 40 transmits the sensor data collected by the sensor data integration management unit 73 to the external device from which access is made (S27).

The external device displays a list of sensor data transmitted from the mobile phone terminal 11 and accepts information about a living support instruction to be transmitted to the mobile phone terminal 11. When the information about a living support instruction to be transmitted to the mobile phone terminal 11 is input by a user, the external device transmits the input information about a living support instruction to the mobile phone terminal 11 (S28). The external device transmits an API execution instruction indicating execution of the designated living support to the electrical home appliance 51.

For example, when it is determined by a user that the room temperature and humidity received as the sensor data are too high, the external device transmits information about a living support instruction containing the description indicating reduction of the temperature of an air conditioner to the mobile phone terminal 11. Further, when a user determines that water intake is too low based on the water server usage data or the health care equipment data, the external device transmits information about a living support instruction containing a message suggesting to drink water to a family member living in a distant place or the like to the mobile phone terminal 11.

When the access management unit 40 of the mobile phone terminal 11 receives the information about a living support instruction from the external device, it determines the description of control of the electrical home appliance 51 (S29). The access management unit 40 then executes control of the electrical home appliance 51 according to the determined description of control (S30). For example, when information indicating reduction of the temperature of an air conditioner is notified as the information about a living support instruction, the access management unit 40 accesses the air conditioner through the local area communication unit 30 and makes control to reduce the set temperature. Further, when a message suggesting to drink water is notified as the information about a living support instruction, the access management unit 40 makes control to display the message "drink more water" on a display device in the home, such as a display connected to a television, a PC device or the like. Alternatively, it may make control to display the message "drink more water" on a display unit of the mobile phone terminal 11.

As described above, with use of the remote control system according to the second exemplary embodiment of the invention, the mobile phone terminal 11 can respond to a request for transmitting sensor data made by a person living in an area distant from the mobile phone terminal 11, and it is thereby possible to realize more appropriate support advice services.

Further, in the processing of Step S26 and Step S27 described above, the sensor data integration management unit 73 may integrate the collected sensor data and transmit information indicating that "it is necessary to reduce the temperature of the air conditioner" and "it is necessary to drink water" to the external device based on the collected data. In other words, the sensor data integration management unit 73 may analyze the collected sensor data and transmit a result of analysis to the external device.

Furthermore, instead of Step S27 described above, Web contents that summarize sensor data or an analysis result of sensor data may be created in the terminal server of the access management unit 40, so that the sensor data or the analysis result of sensor data can be checked by accessing the terminal server from a browser of a personal computer or the like. A user of the external device can thereby check the sensor data or the analysis result of sensor data at any convenient timing.

Further, when a family member living in a distant area is in trouble without knowing how to use the mobile phone terminal, how to view a digital terrestrial broadcasting program with a television compatible with digital broadcasting or make a programmed recording, and how to transmit content data to a stationary television, for example, a person who respond to consultation by telephone or email can view the viewing history of digital terrestrial broadcasting within the mobile phone terminal and make a programmed recording of the next week's program.

The present invention is not restricted to the above-described exemplary embodiments, and various changes and modifications may be made without departing from the scope of the invention.

While the invention has been particularly shown and described with reference to exemplary embodiments thereof, the invention is not limited to these embodiments. It will be understood by those of ordinary skill in the art that various changes in form and details may be made therein without departing from the spirit and scope of the present invention as defined by the claims.

This application is based upon and claims the benefit of priority from Japanese patent application No. 2011-008559, filed on January 19, 2011, the disclosure of which is incorporated herein in its entirety by reference.

### Reference Signs List

- 10: MOBILE COMMUNICATION DEVICE
- 11: MOBILE PHONE TERMINAL
- 20: PUBLIC NETWORK COMMUNICATION UNIT
- 30: LOCAL AREA COMMUNICATION UNIT
- 40: ACCESS MANAGEMENT UNIT
- 50: SENSOR DEVICE
- 51: HOME APPLIANCE
- 52: COMMUNICATION UNIT
- 53: SENSOR MANAGEMENT UNIT
- 54: SENSOR
- 60: PUBLIC NETWORK
- 61: EXTERNAL SERVER
- 62: MOBILE PHONE TERMINAL
- 63: PC TERMINAL
- 71: SENSOR
- 72: MOBILE SENSOR MANAGEMENT UNIT
- 73: SENSOR DATA INTEGRATION MANAGEMENT UNIT
- 74: SENSOR INFORMATION STORAGE UNIT

## Claims

1. A mobile communication device comprising:
public network communication means for wirelessly communicating with a public network;
local area communication means for communicating with a sensor device located in a neighbor area; and
access management means for accepting access from the sensor device through the local area communication means and transmitting sensor device usage information to an external device connected to the public network based on information transmitted from the sensor device, or accepting access from the external device through the public network communication means and controlling operation of the sensor device in accordance with remote control information from the external device.

2. The mobile communication device according to Claim 1, further comprising:
sensor data integration management means for generating the sensor device usage information by combining information transmitted from a plurality of sensor devices,
wherein the access management means transmits the sensor device usage information generated in the sensor data integration management means to the external device connected to the public network.

3. The mobile communication device according to Claim 2, wherein the sensor data integration management means generates the sensor device usage information by combining information transmitted from the sensor device and sensor information detected in a sensor device built in the mobile communication device.

4. The mobile communication device according to any one of Claims 1 to 3, wherein the access management means transmits the sensor device usage information by executing API designated from the sensor device, and controls operation of the sensor device by executing API designated from the external device.

5. The mobile communication device according to any one of Claims 1 to 4, wherein the access management means controls timing to transmit the usage device information to the external device in accordance with remote control from the external device.

6. The mobile communication device according to Claim 2, wherein the sensor data integration management means stores the sensor device usage information and transmits the stored sensor device usage information to the external device in accordance with remote control from the external device.

7. A communication method in a mobile communication device that communicates with an external device connected to a public network and a sensor device located in a neighbor area, comprising:
accepting access from the sensor device and
transmitting sensor device usage information to the external device connected to the public network based on information transmitted from the sensor device; or
accepting access from the external device and
controlling operation of the sensor device in accordance with remote control information from the external device.

8. The communication method according to Claim 7, wherein, when transmitting the sensor device usage information to the external device, the sensor device usage information is generated by combining information transmitted from a plurality of sensor devices.

9. The communication method according to Claim 8, wherein, when generating the sensor device usage information, the sensor device usage information is generated by combining information transmitted from the sensor device and sensor information detected in a sensor device built in the mobile communication device.

10. The communication method according to any one of Claims 7 to 9, wherein, when transmitting the sensor device usage information to the external device, the sensor device usage information is transmitted by executing API designated from the sensor device, or operation of the sensor device is controlled by executing API designated from the external device.

11. The communication method according to any one of Claims 7 to 10 wherein, when transmitting the sensor device usage information to the external device, timing to transmit the usage device information to the external device is controlled in accordance with remote control from the external device.

12. The communication method according to Claim 8, wherein, when generating the sensor device usage information, the sensor device usage information is stored in the mobile communication device, and the stored sensor device usage information is transmitted to the external device in accordance with remote control from the external device.

13. A sensor network system including an external device connected to a public network, a mobile communication device that wirelessly communicates with the public network, and a sensor device located in a neighbor area of the mobile communication device, wherein
the sensor device accesses the mobile communication device for transmitting detected sensor data,
the external device accesses the mobile communication device for transmitting remote control information to remotely control the sensor device, and
the mobile communication device accepts access from the sensor device and transmits sensor device usage information to the external device connected to the public network based on the sensor data transmitted from the sensor device, or accepts access from the external device through the public network communication means and controls operation of the sensor device in accordance with the remote control information from the external device.
